# FASCICULE DE BREVET EUROPEEN

(11) **EP 0 366 524 B1**
(45) Date de publication et mention de la délivrance du brevet: **26.05.1993**
(21) Numéro de dépôt: 89402868.7
(22) Date de dépôt: 17.10.1989
(51) Int. Cl.: A61M 16/04

(54) **Appareil d'assistance respiratoire**
Beatmungsgerät
Respiratory assistance apparatus

(30) Priorité: 26.10.1988 FR 8813978
(43) Date de publication de la demande: 02.05.1990
(73) Titulaire: Boussignac, Georges, F-92160 Antony (FR); Labrune, Jean-Claude, F-92160 Antony (FR)
(72) Inventeur: Boussignac, Georges, F-92160 Antony (FR); Labrune, Jean-Claude, F-92160 Antony (FR)
(74) Mandataire: Bonnetat, Christian

(56) Documents cités:
- EP-A- 0 074 809
- EP-A- 0 245 142
- DE-A- 3 327 342
- GB-A- 2 114 896
- US-A- 4 285 340

## Description

La présente invention a pour objet un appareil d'assistance respiratoire, utilisable sur des patients dont la respiration spontanée est absente ou insuffisante.

On connaît déjà des appareils d'assistance respiratoire voir DE-B-3327342 comportant des moyens pour amener du gaz respiratoire dans les poumons d'un patient et, éventuellement, pour en évacuer ledit gaz, par l'intermédiaire d'un tube d'assistance respiratoire dont l'extrémité distale est généralement destinée à être introduite dans la trachée du patient. Dans ce cas, il est d'usage de disposer, à l'extrémité distale de la sonde, un ballonnet gonflable. Il existe donc un risque de surpression dans les poumons du patient, si l'alimentation de gaz n'est pas parfaitement contrôlée, surpression qui peut, bien sûr, se révéler dangereuse pour le patient.

L'invention a pour but de remédier à cet inconvénient, et concerne un appareil d'assistance respiratoire du type indiqué précédemment, grâce auquel tout risque de surpression dans les poumons peut être évité et tel que défini dans la revendication 1.

Ainsi, toute surpression à l'intérieur du ballonnet traduira une surpression, ou tout au moins un risque de surpression, dans les poumons, et l'alimentation en gaz respiratoire du patient pourra être réglée en conséquence. De plus, la détection de variations de pression dans le ballonnet permet d'éviter tout risque de surpression à l'intérieur de celui-ci, ce qui pourrait avoir pour conséquence l'endommagement de la paroi de la trachée sur laquelle il s'appuie.

Selon une autre caractéristique de l'invention, l'appareil comprend des seconds moyens de détection de la pression en aval dudit ballonnet. On peut ainsi détecter la pression "vraie" régnant à l'intérieur des poumons.

Avantageusement, lesdits premiers moyens de détection sont constitués par un capteur de pression relié au conduit d'alimentation de gaz du ballonnet.

En particulier, une soupape de sécurité peut être reliée audit conduit d'alimentation de gaz du ballonnet. On peut, ainsi, si nécessaire, dégonfler rapidement le ballonnet.

Par ailleurs, lesdits seconds moyens de détection peuvent être constitués par un capteur de pression relié à un conduit débouchant, en aval dudit ballonnet, à l'extrémité distale dudit tube.

Selon une autre caractéristique de l'invention, la partie d'extrémité distale du tube comporte un premier capillaire, logé dans la paroi de celle-ci, relié auxdits premiers moyens de détection et débouchant dans ledit ballonnet.

Avantageusement, la partie d'extrémité distale du tube comporte un deuxième capillaire, logé dans la paroi de celle-ci, relié auxdits seconds moyens de détection et débouchant en aval du ballonnet.

De plus, la partie d'extrémité distale du tube peut comporter une pluralité de troisièmes capillaires, logés dans la paroi de celle-ci, pour l'alimentation en gaz respiratoire du patient. De préférence, l'extrémité débouchant dans ledit tube de chacun desdits troisièmes capillaires est préformée. Le préformage de ces capillaires consiste en une préformation dont la forme rappelle la forme générale ainsi que les variations de section d'un venturi. Ce préformage spécifique permet l'optimisation des performances dynamiques des fluides respiratoires dans le cadre d'une régulation continue des débits, qualité des mélanges, des pressions d'insufflation par rapport à la compliance pulmonaire.

Par ailleurs, l'appareil peut comprendre une électronique adaptée pour piloter l'alimentation en gaz respiratoire du patient, ainsi que le gonflage dudit ballonnet, en fonction d'informations transmises par lesdits premiers et seconds moyens de détection de pression.

Les figures du dessin annexé feront bien comprendre comment l'invention peut être réalisée.

La figure 1 est un schéma synoptique de l'appareil d'assistance respiratoire selon l'invention.

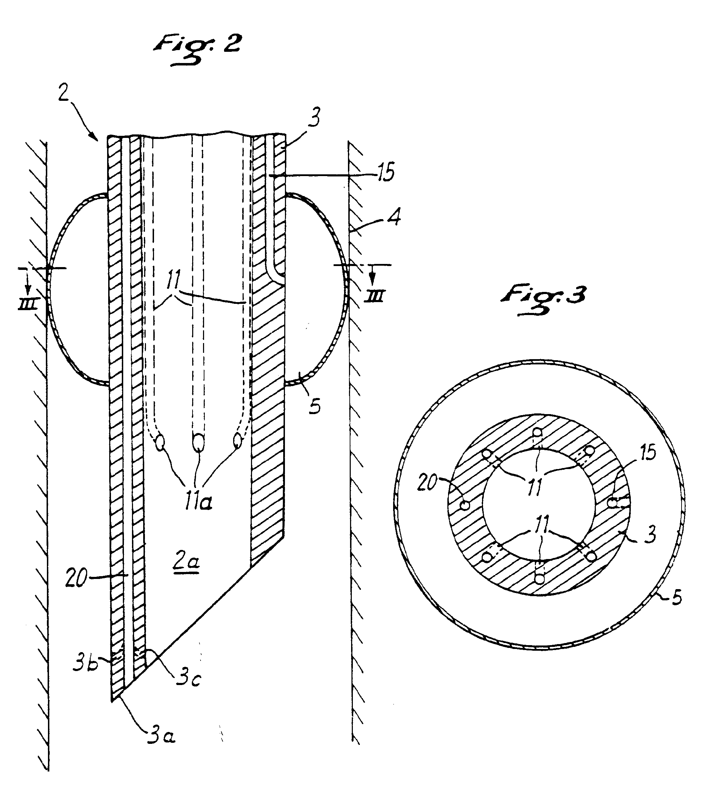

La figure 2 est une coupe longitudinale de la partie d'extrémité distale de la sonde d'intubation.

La figure 3 est une coupe selon la ligne III-III de la figure 2.

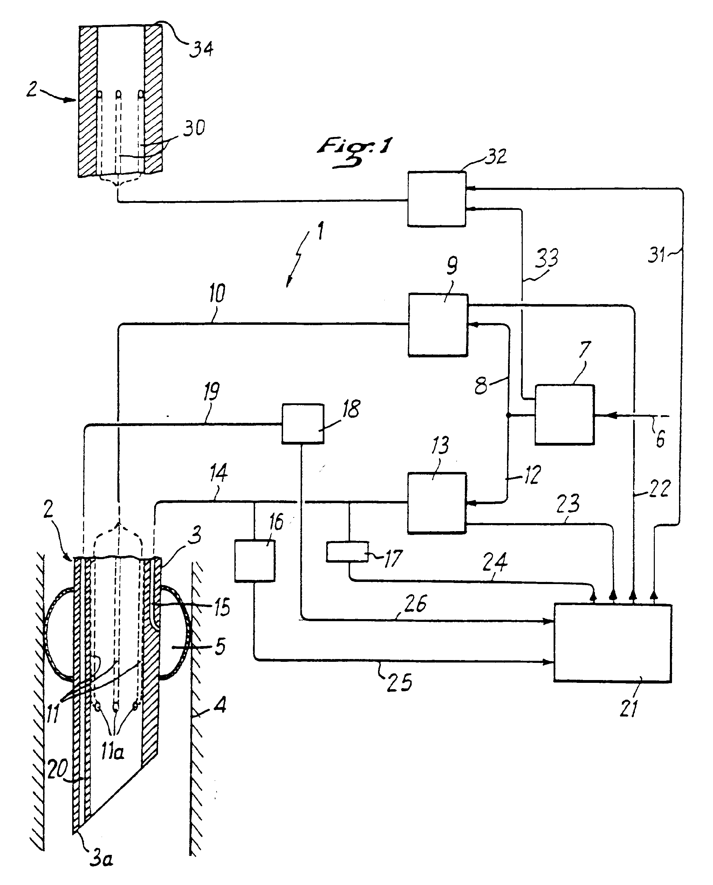

En se référant notamment à la figure 1, l'appareil d'assistance respiratoire 1 comprend un tube d'assistance respiratoire 2 dont l'extrémité distale 3 destinée à être introduite dans la trachée 4 d'un patient est munie d'un ballonnet gonflable 5. Ce ballonnet 5 a pour but de maintenir l'extrémité 3 du tube en position dans la trachée 4 et d'éviter le "retour" non contrôlé du gaz respiratoire par la trachée.

Par ailleurs, une arrivée 6 de gaz respiratoire sous pression, qui peut être de l'oxygène ou un mélange de gaz contenant de l'oxygène, est reliée à un régulateur de pression 7 délivrant une pression de sortie de, par exemple, 3 bars. Le régulateur de pression 7 est, à son tour, relié, par un conduit 8, à des moyens de commande 9 de l'alimentation en gaz respiratoire du patient. Ces moyens de commande peuvent être constitués d'électrovannes commandant les périodes insufflatoires et éventuellement expiratoires de l'assistance respiratoire du patient. De plus, les moyens de commande 9 sont reliés, par l'intermédiaire d'un conduit 10, à une pluralité de capillaires 11, logés dans la paroi de la partie d'extrémité distale 3 du tube 2, pour l'alimentation en gaz respiratoire du patient, et dont l'extrémité 11a débouchant dans le tube est préformée.

De plus, le régulateur de pression 7 est relié, par un conduit 12, à des moyens de commande 13 du gonflage du ballonnet 5, eux-mêmes reliés, par un conduit 14, à un capillaire 15, logé dans la paroi de la partie d'extrémité distale 3 du tube et débouchant dans le ballonnet 5.

Par ailleurs, un capteur de pression 16 est relié au conduit 14 d'alimentation de gaz du ballonnet 5, de même qu'une soupape de sécurité 17. Egalement, il est prévu un capteur de pression 18 relié, par un conduit 19, à un capillaire 20, logé dans la paroi de la partie d'extrémité distale 3 du tube, débouchant en aval du ballonnet 5 et, avantageusement, comme montré sur le dessin, à la pointe 3a de l'extrémité 3 du tube 2. Le capillaire 20 pourra également déboucher en 3b ou 3c pour des motifs évidents de sécurité, pour éviter des obstructions pouvant être provoquées par des sérosités en provenance de l'appareil respiratoire.

L'appareil d'assistance respiratoire 1 comprend de plus une électronique 21 adaptée pour piloter l'alimentation en gaz respiratoire du patient (liaison 22), ainsi que le gonflage (et éventuellement la purge) du ballonnet 5 (liaisons 23 et 24), en fonction d'informations transmises par les capteurs de pression 16 et 18, par l'intermédiaire des liaisons 25 et 26, respectivement.

L'appareil d'assistance respiratoire 1 peut également comprendre l'asservissement nécessaire à l'alimentation en gaz des capillaires 30 de façon à provoquer une aide expiratoire. L'électronique 21 est adaptée pour piloter l'alimentation en gaz (liaison 31) des capillaires 30 à travers un moyen de commande 32 recevant le gaz du régulateur de pression 7 et d'un conduit d'alimentation 33. Les capillaires 30 sont logés dans la paroi de l'extrémité proximale 34 du tube 2 et permettent de réaliser un entraînement des gaz rejetés par le patient selon les principes de la dynamique des fluides. Comme pour les capillaires 11, l'extrémité des capillaires 30 et de la paroi du tube 2 sont configurés selon des variations de section de type "venturi" afin d'obtenir la qualité d'écoulement recherchée.

L'appareil respiratoire 1 pourra ainsi disposer d'un asservissement du cycle respiratoire complet sous le contrôle des informations de pression 16 et 18. On disposera ainsi d'un appareil muni d'une électronique permettant de régler dans le temps et successivement la durée de l'inspiration et la durée de l'expiration tout en respectant les temps morts souhaités et réglés par l'opérateur. Le contrôle de pression permettra sans difficulté de maintenir une pression positive grâce à une alimentation continue sur un capillaire 11 et ce même en fin du cycle d'expiration. On pourra egalement munir l'appareil respiratoire d'autres sondes de régulation, sonde de mesure d'hygrométrie ou sonde de mesure de débit.

Ainsi, l'appareil d'assistance respiratoire de l'invention permet l'alimentation d'un patient en gaz respiratoire sans risque de surpression dans les poumons de celui-ci, du fait que la pression dans le ballonnet 5 représentative de la pression dans les poumons et la pression "vraie" peuvent être mesurées par les capteurs de pression respectifs 16 et 18, et que, à partir de ces mesures, peut être réglée l'alimentation en gaz respiratoire du patient, ainsi d'ailleurs que le gonflage du ballonnet.

## Revendications

1. Appareil d'assistance respiratoire, comportant un tube d'assistance respiratoire (2) dont l'extrémité distale (3) destinée à être introduite dans la trachée (4) d'un patient est munie d'un ballonnet gonflable (5), des moyens (6-11) pour amener du gaz respiratoire dans les poumons du patient, par l'intermédiaire dudit tube (2), et des premiers moyens (16) de détection des variations de la pression à l'intérieur dudit ballonnet (5),
caractérisé en ce que lesdits premiers moyens de détection (16) sont reliés à une électronique (21) adaptée pour piloter l'alimentation en gaz respiratoire du patient en fonction d'informations transmises par lesdits premiers moyens de détection (16).

2. Appareil selon la revendication 1,
caractérisé en ce qu'il comprend des seconds moyens (18) de détection de la pression en aval dudit ballonnet (5), reliés à ladite électronique (21) adaptée de plus pour piloter l'alimentation en gaz respiratoire du patient en fonction d'informations transmises par lesdits seconds moyens de détection (18).

3. Appareil selon la revendication 1 ou la revendication 2,
caractérisé en ce que lesdits premiers moyens de détection sont constitués par un capteur de pression (16) relié au conduit d'alimentation de gaz (14) du ballonnet (5).

4. Appareil selon la revendication 3,
caractérisé en ce qu'une soupape de sécurité (17) est reliée audit conduit d'alimentation de gaz (14) du ballonnet (5).

5. Appareil selon l'une quelconque des revendications 2 à 4,
caractérisé en ce que lesdits seconds moyens de détection sont constitués par un capteur de pression (18) relié à un conduit (19,20) débouchant, en aval dudit ballonnet (5), à l'extrémité distale (3) dudit tube (2).

6. Appareil selon l'une quelconque des revendications 1 à 5,
caractérisé en ce que la partie d'extrémité distale (3) du tube comporte un premier capillaire (15) logé dans la paroi de celle-ci, relié auxdits premiers moyens de détection (16) et débouchant dans ledit ballonnnet (5).

7. Appareil selon la revendication 6,
caractérisé en ce que la partie d'extrémité distale (3) du tube comporte un deuxième capillaire (20) logé dans la paroi de celle-ci, relié auxdits seconds moyens de détection (18) et débouchant en aval du ballonnet (5).

8. Appareil selon la revendication 6 ou la revendication 7,
caractérisé en ce que la partie d'extrémité distale (3) du tube comporte une pluralité de troisièmes capillaires (11), logés dans la paroi de celle-ci, pour l'alimentation en gaz respiratoire du patient.

9. Appareil selon la revendication 8,
caractérisé en ce que l'extrémité (11a) débouchant dans ledit tube de chacun desdits troisièmes capillaires (11) est préformée.

10. Appareil selon l'une quelconque des revendications 2 à 9,
caractérisé par une électronique (21) adaptée pour piloter l'alimentation en gaz respiratoire du patient, ainsi que le gonflage dudit ballonnet, en fonction d'informations transmises par lesdits premiers (16) et seconds (18) moyens de détection de pression.

## Claims

1. A breathing aid comprising: a breathing aid tube (2) whose distal end (3) introducible into a patient's trachea (4) has an inflatable bag (5); means (6 - 11) for feeding respiratory gas to the patient's lungs through the tube (2); and first means (16) for detecting variations of the pressure in the bag (5), characterised in that the first detecting means (16) are connected to an electronic facility (21) adapted to control the supply of respiratory gas to the patient in dependence upon data transmitted by the first detecting means (16).

2. An apparatus according to claim 1, characterised in that it comprises second means (18) for detecting the pressure downstream of the bag (5), the second means (18) being connected to the electronic facility (21) which is also adapted to control the supply of respiratory gas to the patient in dependence upon data transmitted by the second detecting means (18).

3. An apparatus according to claim 1 or claim 2, characterised in that the first detecting means take the form of a pressure sensor (16) connected to the gas supply duct (14) of the bag (5).

4. An apparatus according to claim 3, characterised in that a safety valve (17) is connected to the gas supply duct (14) of the bag (5).

5. An apparatus according to any of claims 2 to 4, characterised in that the second detecting means take the form of a pressure sensor (18) connected to a duct (19, 20) which extends downstream of the bag (5) to the distal end (3) of the tube (2).

6. An apparatus according to any of claims 1 to 5, characterised in that the distal end part (3) of the tube comprises a first capillary (15) which is received in the wall of the part (3), is connected to the first detecting means (16) and extends into the bag (5).

7. An apparatus according to claim 6, characterised in that the distal end part (3) of the tube comprises a second capillary (20) which is received in the wall of the part (3), is connected to the second detecting means (18) and extends to downstream of the bag (5).

8. An apparatus according to claim 6 or claim 7, characterised in that the distal end part (3) of the tube comprises a number of third capillaries (11) which are received in the wall of the part (3) and which serve to supply respiratory gas to the patient.

9. An apparatus according to claim 8, characterised in that the end (11a) extending into the tube of each of the third capillaries (11) is preshaped.

10. An apparatus according to any of claims 2 to 9, characterised by an electronic facility (21) adapted to control the supply of respiratory gas to the patient and to control inflation of the bag in dependence upon data transmitted by the first pressure-detecting means and second pressure-detecting means (16, 18 respectively).

## Patentansprüche

1. Beatmungsgerät, das einen Beatmungstubus (2) umfaßt, dessen distales Ende (3), das dazu bestimmt ist, in die Luftröhre (4) eines Patienten eingeführt zu werden, mit einem aufblasbaren Ballon (5) ausgestattet ist, das Mittel (6 bis 11) für die Zuführung von Beatmungsgas in die Lunge des Patienten mit Hilfe des Tubus (2) und das erste Mittel (16) zur Erfassung von Druckschwankungen im Innern des Ballons (5) umfaßt, dadurch gekennzeichnet, daß die ersten Erfassungsmittel (16) mit einer Elektronik (21) verbunden sind, die so ausgebildet ist, daß sie die Beatmungsgasversorgung eines Patienten als Funktion der von den ersten Erfassungsmitteln (16) übermittelten Informationen steuert.

2. Gerät nach Anspruch 1 dadurch gekennzeichnet, daß es zweite Druckerfassungsmittel (18) auf der stromabwärtigen Seite des Ballons (5) umfaßt, die mit der Elektronik (21) verbunden sind, die zusätzlich so ausgebildet ist, daß sie die Beatmungsgasversorgung eines Patienten als Funktion der von den zweiten Erfassungsmitteln (18) übermittelten Informationen steuert.

3. Gerät nach Anspruch 1 oder Anspruch 2, dadurch gekennzeichnet, daß die ersten Erfassungsmittel von einem Druckaufnehmer (16) gebildet sind, der mit der Gaszuführungsleitung (14) des Ballons (5) verbunden ist.

4. Gerät nach Anspruch 3, dadurch gekennzeichnet, daß ein Sicherheitsventil (17) mit der Gaszuführungsleitung (14) des Ballons (5) verbunden ist.

5. Gerät nach einem der Ansprüche 2 bis 4, dadurch gekennzeichnet, daß die zweiten Erfassungsmittel von einem Druckaufnehmer (18) gebildet sind, der mit einer Leitung (19, 20) verbunden ist, die stromabwärtig vom Ballon (5) am distalen Ende (3) des Tubus (2) ausmündet.

6. Gerät nach einem der Ansprüche 1 bis 5, dadurch gekennzeichnet, daß der distale Endbereich (3) des Tubus eine erste Kapillare 15 umfaßt, die sich in dessen Seitenwand befindet, die mit den ersten Erfassungsmitteln (16) verbunden ist und die in den Ballon (5) mündet.

7. Gerät nach Anspruch 6, dadurch gekennzeichnet, daß der distale Endbereich 3 des Tubus eine zweite Kapillare (20) umfaßt, die sich in dessen Wand befindet, die mit den zweiten Erfassungsmitteln (18) verbunden ist und die stromabwärtig vom Ballon (5) ausmündet.

8. Gerät nach Anspruch 6 oder Anspruch 7, dadurch gekennzeichnet, daß der distale Endbereich (3) des Tubus eine Mehrzahl dritter, in dessen Wand befindlicher Kapillaren (11) für die Beatmungsgasversorgung des Patienten umfaßt.

9. Gerät nach Anspruch 8, dadurch gekennzeichnet, daß das Ende (11a) jeder dritten Kapillare (11), das im Tubus ausmündet, vorgeformt ist.

10. Gerät nach einem der Ansprüche 2 bis 9, gekennzeichnet durch eine Elektronik (21) die so ausgebildet ist, daß sie die Beatmungsgasversorgung des Patienten ebenso wie das Aufblasen des Ballons als Funktion der von den ersten (16) bzw. zweiten (18) Druckerfassungsmitteln übermittelten Informationen steuert.
